Europäisches Patentamt

European Patent Office

Office européen des brevets

(11) Publication number: **0 264 516**
**A1**

# EUROPEAN PATENT APPLICATION

(21) Application number: 86850363.2

(22) Date of filing: 21.10.86

(51) Int. Cl.4: **A61F 5/56**

(43) Date of publication of application:
27.04.88 Bulletin 88/17

(84) Designated Contracting States:
AT BE CH DE FR GB IT LI LU NL

(71) Applicant: **Lundberg, Siw**
**Rälambsvägen 19**
**S-112 59 Stockholm(SE)**

(72) Inventor: **Lundberg, Siw**
**Rälambsvägen 19**
**S-112 59 Stockholm(SE)**

(74) Representative: **Kransell, Rolf Arne Steinert et al**
**Kransell & Wennborg AB Sandhamnsgatan 42**
**S-115 28 Stockholm(SE)**

(54) Anti-snoring device.

(57) The invention relates to an anti-snoring device and is based on the realization that snoring may be counteracted by means of a tongue-like member (2) contacting the front portion of the soft palate (M) which, by said contact, is prevented from carrying out vibrating movements generating the snoring sounds.

Fig. 4

EP 0 264 516 A1

## Anti-snoring device

Snoring is a phenomenon which to many people is very disturbing and in some cases even injurious to the health. It has a higher frequency with men than with women and the tendency to snore increases with age.

One obvious inconvenience is the acoustic disturbances to which individuals sleeping in the same room as the snoring person are exposed. However, in later years, it has become more and more clear that the snoring person is medically affected by this complaint. One such consequence is that the disturbances of the breathing which the snoring generates result in a reduced ventilation. This does negatively affect the natural exchange of gases. There will be a too low supply of oxygen to the body organs which principally affects the brain. Further, the carbon-dioxide content of the blood is increased. In serious cases disturbances of this type lead to so-called apne attacks.

For the just-mentioned reasons efforts have been made to develop devices capable of preventing, or at least counteracting, snoring. The corresponding efforts have started from the assumption that - since experience shows that a snoring person normally rests on his back - the snoring sounds would be the result of the tongue sliding backwards down into the pharynx thereby making breathing more difficult by partly blocking the air passage. If the blocking becomes substantial, the snoring person wakes up. For those reasons attempts have been made to prevent the tongue from sliding backwards. U.S. 3 132 647 discloses a device of this type.

The present invention is based on the realization that the primary cause of snoring is not the one just mentioned but a quite different one, namely vibrations of the soft palate. Practical experiments have shown that this theory is correct, meaning that snoring can be counteracted if the soft palate is prevented from vibrating. A device according to the invention is mainly characterized in that it comprises a tongue-like member which is supported by a holder and which is oriented in such a way and extends that far back into the oral cavity that it will contact the soft palate.

One embodiment of the invention will now be described with reference to the drawing.

Figure 1 does diagrammatically show a vertical section, taken along a central plane, through the oral cavity, the nasal cavity and adjacent tissues.

Figure 2 is a perspective view showing a device according to the described embodiment.

Figure 3 is a front view of the oral cavity and the pharynx, the mouth being wide open and the subject of the invention in its installed position.

Figure 4 corresponds to Figure 1 but differs therefrom that it does also include the device according to the invention placed in position for use.

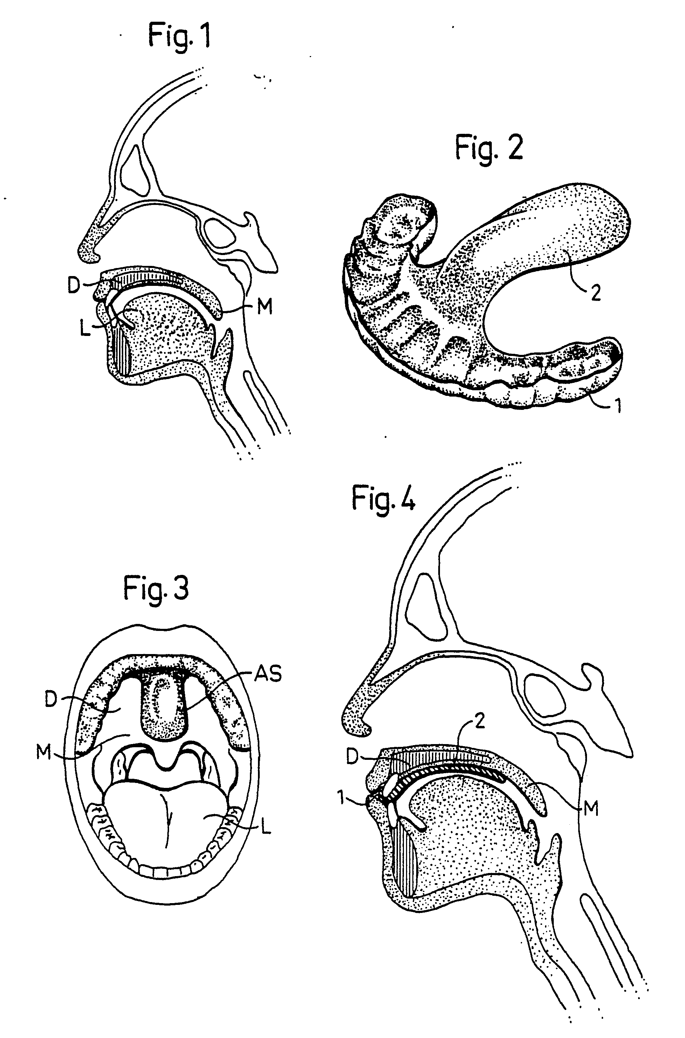

In Figure 1 D designates the hard palate, M the soft palate and L the tongue. The same reference letters are used in Figure 3 where AS refers to the anti-snoring device, shown in an isometric view in Figure 2. The device has two main parts, a holder 1 and the tongue-like contact member 2. According to the illustrated embodiment the holder is a type of bite splint having essentially the same structure as the dental protections used in ice-hockey, boxing and other tough sports. However, as an alternative, it could be a dental plate for the upper jaw in which case the contact member could be detachably fixed to the holder but in the embodiment illustrated the holder and the contact member form an integral unit, e.g. made of an acrylic resin.

The vertical section of Figure 4 illustrates that the free end of member 2 contacts the front part of the soft palate M. It is a special advantage of the invention that such a limited contact between the soft palate and member 2 is sufficient to prevent vibrations of the soft palate. The reason for this is that, in the context discussed, the soft palate may be considered equivalent to a pendulum in combination with the fact that the swinging movement of a pendulum can be inhibited by the application of a lateral force adjacent its oscillating axis. Also, as is illustrated in Figures 2 and 3, the tongue-like contact member is relatively narrow. Both these facts, its lesser extent longitudinally and laterally as compared with the palate, mean that the device can be used without in any way to inconvenience the person using it. A particularly valuable advantage of the limited extent is that there will be no contact with those reflex areas in the palate which may generate nauseating or vomiting reflexes.

### Claims

1. A device preventing or reducing snoring and adapted to be installed in the oral cavity of a snoring person, **characterized** in that it consists of a tongue-like member (2) which is supported by a holder (1) and which in its installed position is arranged to contact the soft palate.

2. A device as claimed in Claim 1, **characterized** in that its holder (1) is constituted by a bite splint.

3. A device as claimed in Claim 1 or 2, **characterized** in that the contact member (2) and the holder (1) form an integral unit.

4. A device as claimed in Claim 1, **characterized** in that the contact member (2) is adapted for detachable mounting at an upper jaw prothesis.

Fig. 1

Fig. 2

Fig. 3

Fig. 4

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | US-A-3 818 906 (STUBBS) * Whole document * | 1 | A 61 F 5/56 |
| Y | | 2-4 | |
| D,Y | US-A-3 132 647 (CORNIELLO) * Claim 1; column 2, line 70 - column 3, line 3 * | 2-4 | |
| A | GB-A-1 472 067 (ELLIS) | | |
| A | US-A-3 976 054 (EVANS) | | |
| E | US-A-4 669 459 (SPIEWAK) * Figures; abstract * | 1 | |

TECHNICAL FIELDS SEARCHED (Int. Cl.4)

A 61 F
A 61 B
A 63 B

The present search report has been drawn up for all claims

| Place of search | Date of completion of the search | Examiner |
|---|---|---|
| THE HAGUE | 08-09-1987 | STEENBAKKER J. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03.82